# EUROPEAN PATENT APPLICATION

(11) **EP 4 509 090 A1**
(43) Date of publication of application: **19.02.2025**
(21) Application number: 23788087.7
(22) Date of filing: 10.03.2023
(51) Int. Cl.: A61B 90/70

(54) **REMOVAL TOOL AND REMOVAL SYSTEM**

(30) Priority: 11.04.2022 JP 2022065128
(71) Applicant: HOYA CORPORATION, Shinjuku-ku Tokyo 160-8347 (JP)
(72) Inventor: SUGITA, Noriyuki, Tokyo 160-8347 (JP)
(74) Representative: Schaumburg und Partner Patentanwälte mbB
(86) International application number: PCT/JP2023/009206
(87) International publication number: WO 2023/199665

(57) **Abstract**

For purpose of providing a removal tool (1) and a removal system with high operability in which the removal tool (1) is manually set in a channel (2B) of a precise and small endoscopic treatment tool (2) and removes an adhering substance adhered to an inside of the channel (2B), the removal tool (1) for removing the adhering substance adhered to the inside of the channel (2B) of the endoscopic treatment tool (2) is configured to include: a bottomed cylindrical body (10) having an opening at one end; and an elongated part (20) formed in a hollow portion (12) of the bottomed cylindrical body (10) so as to extend from a bottom (12B) of the hollow portion (12) toward an opening (12A) side of the hollow portion (12), which is capable of inserting into the channel (12B), and the hollow portion (12) of the bottomed cylindrical body (10) expands from the bottom (12B) side toward the opening (12A).

## Description

### Technical Field

The present invention relates to a removal tool and a removal system.

### Background Art

In an endoscopic submucosal dissection (ESD), an endoscopic treatment tool provided with a high frequency knife for excising a mucous membrane or the like by energizing a high frequency current is used.

In this type of endoscopic treatment tool, for example, hemoglobin, protein, or the like thermally coagulated by cauterization with the high frequency knife may adhere to an inside of a channel of the endoscopic treatment tool. Thus, there is a possibility that the channel is clogged by the adhering substance. A worker, therefore, removes the adhering substance from the inside of the channel using, for example, a removal tool (see, e.g., Patent Literature 1).

### Citation List

### Patent Literature

Patent Literature 1: US 2020/0323610 A

### Summary of Invention

### Technical Problem

The worker manually sets the removal tool in the channel of the precise and small endoscopic treatment tool to remove the adhering substance adhered to the inside of the channel. The removal tool, therefore, is required to have high operability.

The present invention has been made in view of the above circumstances, and an object of the present invention is to provide a removal tool and a removal system having high operability.

### Solution to Problem

A removal tool according to an embodiment of the present invention is provided for removing an adhering substance adhered to an inside of a channel of an endoscopic treatment tool, and the removal tool includes: a bottomed cylindrical body having an opening at one end; and an elongated part formed in a hollow portion of the bottomed cylindrical body so as to extend from a bottom of the hollow portion toward an opening side of the hollow portion, which is capable of inserting into the channel. The hollow portion of the bottomed cylindrical body expands from the bottom side toward the opening.

In the removal tool according to an embodiment of the present invention, a plurality of slits dividing a side surface of the bottomed cylindrical body into a plurality of portions may be formed in a circumferential direction around an axis of the bottomed cylindrical body. In such a configuration, when the endoscopic treatment tool is inserted into and pressed against the hollow portion, a portion of the bottomed cylindrical body located between the slits is elastically deformed, which causes the hollow portion to expand from the bottom side toward the opening.

The removal tool according to an embodiment of the present invention may further includes an exterior part holding the bottomed cylindrical body. In such a configuration, for example, the bottomed cylindrical body has higher pliability than the exterior part.

In an embodiment of the present invention, the hollow portion of the bottomed cylindrical body may include a tapered portion expanding continuously or stepwise from the bottom side toward the opening.

In an embodiment of the present invention, the bottomed cylindrical body includes a constricted portion formed, for example, in a cylindrical shape and having an outer diameter narrower at a central portion than at end portions in an axial direction of the bottomed cylindrical body.

In an embodiment of the present invention, the bottomed cylindrical body is formed of a material through which light having at least a part of visible wavelength is capable of passing.

In an embodiment of the present invention, an edge portion may be formed on a side surface of the elongated part.

In an embodiment of the present invention, a distal end portion of the elongated part has, for example, a tapered shape.

In an embodiment of the present invention, the hollow portion of the bottomed cylindrical body may have a circular cross section orthogonal to an axial direction of the bottomed cylindrical body, and the elongated part may be disposed in the hollow portion so as to pass through a center of the circular cross section and extend along the axial direction.

In an embodiment of the present invention, the hollow portion of the bottomed cylindrical body may have a circular cross section orthogonal to an axial direction of the bottomed cylindrical body, and the elongated part may be disposed in the hollow portion so as to pass through a position deviated from a center of the circular cross section and extend along the axial direction.

A removal system according to an embodiment of the present invention includes: any one of the removal tools described above; and an endoscopic treatment tool to be inserted into the removal tool. In the removal system, an indicator indicating an amount of the endoscopic treatment tool to be inserted into the removal tool is marked on the endoscopic treatment tool.

A removal tool according to an embodiment of the present invention is provided for removing an adhering substance adhered to an inside of a channel of an endoscopic treatment tool, and the removal tool includes: a bottomed cylindrical body having an opening at one end; and an elongated part formed in a hollow portion of the bottomed cylindrical body so as to extend from a bottom of the hollow portion toward an opening side of the hollow portion, which is capable of inserting into the channel. The hollow portion of the bottomed cylindrical body has a circular cross section orthogonal to an axial direction of the bottomed cylindrical body, and the elongated part is disposed in the hollow portion so as to pass through a position deviated from a center of the circular cross section and extend along the axial direction.

### Advantageous Effects of Invention

According to an embodiment of the present invention, the removal tool and the removal system having high operability are provided.

### Brief Description of Drawings

Fig. 1 is a perspective view of a removal tool according to a first embodiment of the present invention.
Fig. 2 is an arrow view of the removal tool according to the first embodiment of the present invention as viewed from a direction of an arrow A in Fig. 1.
Fig. 3 is a cross-sectional view of the removal tool according to the first embodiment of the present invention.
Fig. 4 is an overall view of an endoscopic treatment tool inserted into the removal tool in the first embodiment of the present invention.
Fig. 5 is a view illustrating an internal structure of a distal end portion of the endoscopic treatment tool inserted into the removal tool in the first embodiment of the present invention.
Fig. 6 is a view illustrating a state in which the endoscopic treatment tool is inserted into the removal tool in the first embodiment of the present invention.
Fig. 7 is a view illustrating a state in which an adhering substance adhered to an inside of a channel of the endoscopic treatment tool is removed in the first embodiment of the present invention.
Fig. 8 is a perspective view of a removal tool according to a second embodiment of the present invention.
Fig. 9 is a perspective view illustrating an internal structure according to the second embodiment of the present invention.
Fig. 10 is an exploded perspective view illustrating the internal structure according to the second embodiment of the present invention.
Fig. 11 is a cross-sectional view illustrating the internal structure according to the second embodiment of the present invention.
Fig. 12A is a view illustrating a state in which an endoscopic treatment tool is inserted into the removal tool in the second embodiment of the present invention.
Fig. 12B is a view illustrating a state in which the endoscopic treatment tool is inserted into the removal tool in the second embodiment of the present invention.
Fig. 13 is a perspective view of a removal tool according to a third embodiment of the present invention.
Fig. 14 is a cross-sectional view illustrating an internal structure of the removal tool according to the third embodiment of the present invention.
Fig. 15A is a view illustrating a state in which an endoscopic treatment tool is inserted into the removal tool in the third embodiment of the present invention.
Fig. 15B is a view illustrating a state in which the endoscopic treatment tool is inserted into the removal tool in the third embodiment of the present invention.
Fig. 16 is a cross-sectional view illustrating an internal structure of a removal tool according to a fourth embodiment of the present invention.
Fig. 17 is a cross-sectional view taken from line F-F in Fig. 16.
Fig. 18 is a view illustrating a state in which an endoscopic treatment tool is inserted into the removal tool in the fourth embodiment of the present invention.
Fig. 19A is a view illustrating a state in which the endoscopic treatment tool is inserted into the removal tool in the fourth embodiment of the present invention.
Fig. 19B is a view illustrating a state in which the endoscopic treatment tool is inserted into the removal tool in the fourth embodiment of the present invention.
Fig. 19C is a view illustrating a state in which the endoscopic treatment tool is inserted into the removal tool in the fourth embodiment of the present invention.
Fig. 19D is a view illustrating a state in which the endoscopic treatment tool is inserted into the removal tool in the fourth embodiment of the present invention.
Fig. 20A is a view illustrating an XY cross section of a rod part according to a first modification of the fourth embodiment of the present invention.
Fig. 20B is a view illustrating an XY cross section of a rod part according to a second modification of the fourth embodiment of the present invention.

### Description of Embodiments

Hereinafter, a removal tool and a removal system according to an embodiment of the present invention will be described with reference to the drawings.

The removal tool according to the present embodiment removes an adhering substance adhered to an inside of a channel of an endoscopic treatment tool. The removal system includes the removal tool and the endoscopic treatment tool. A work of removing the adhering substance using the removal tool is performed by, for example, an operator (doctor) or another medical worker (physician assistant, nurse, etc.). A person who performs the work of removing the adhering substance using the removal tool is collectively referred to as a worker.

### [First Embodiment]

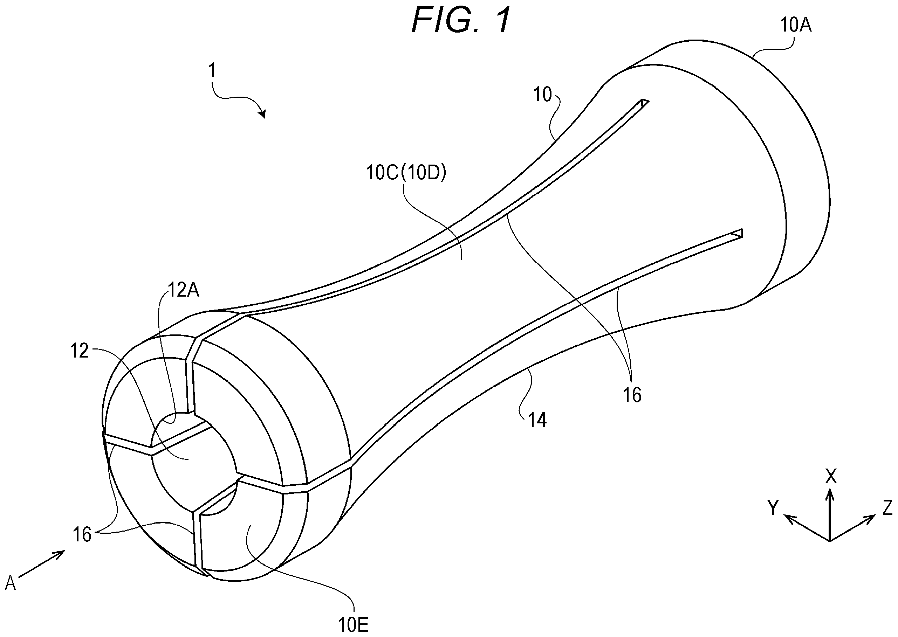

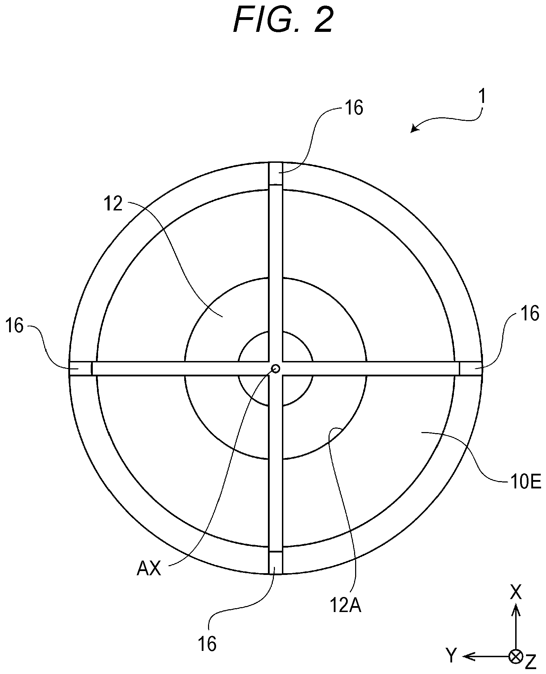

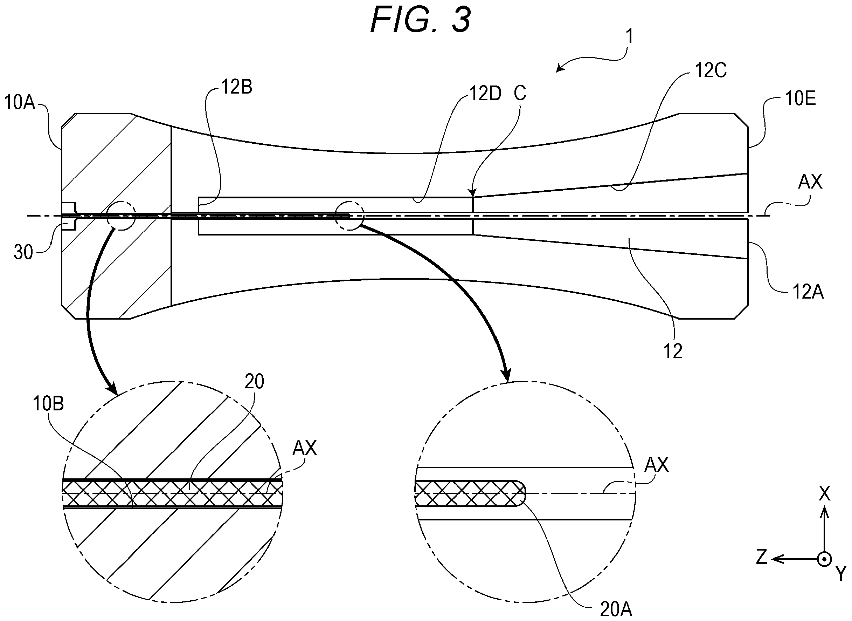

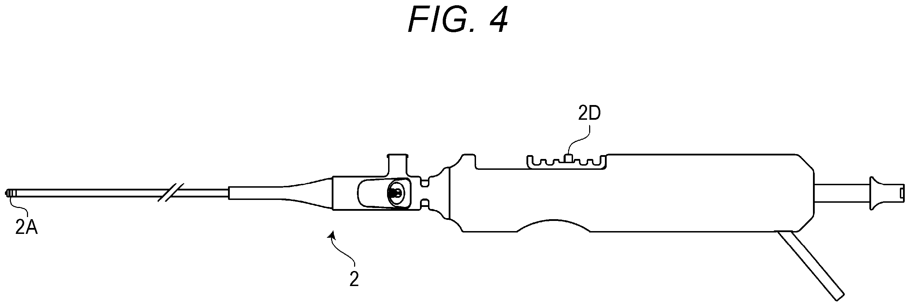

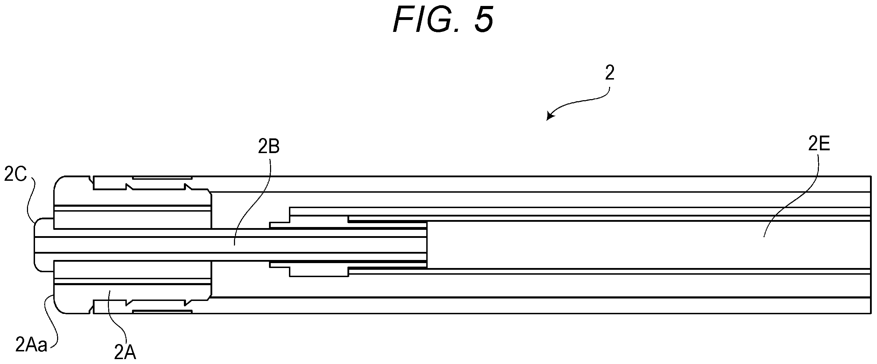

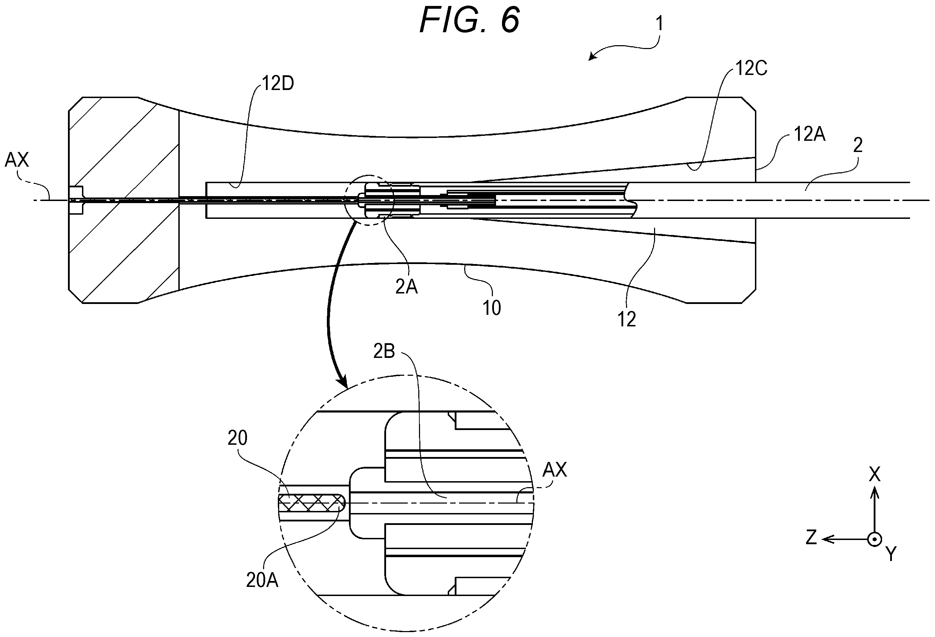

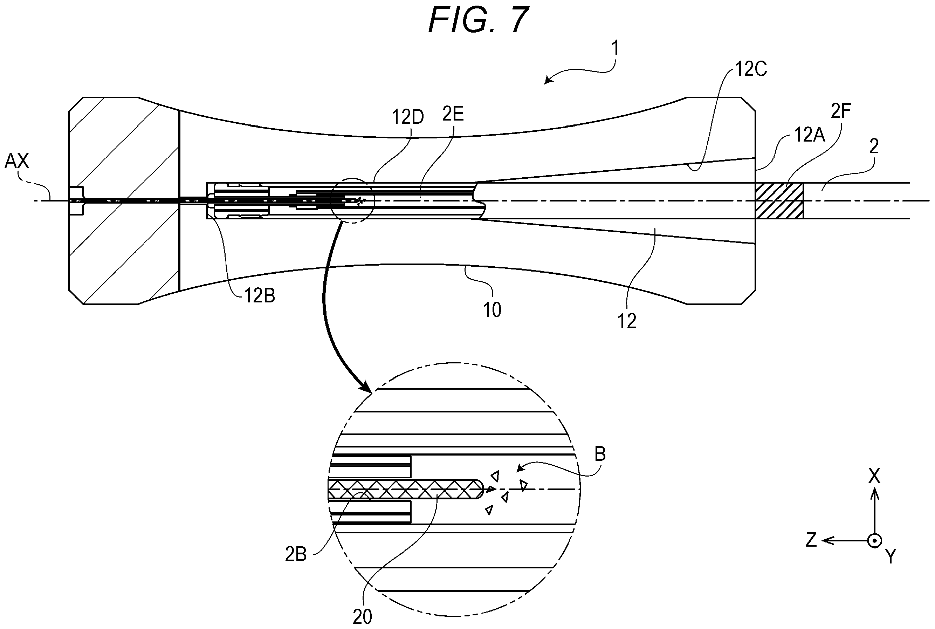

Fig. 1 is a perspective view of a removal tool 1 according to a first embodiment of the present invention. Fig. 2 is an arrow view of the removal tool 1 as viewed from a direction of an arrow A in Fig. 1. Fig. 3 is a cross-sectional view of the removal tool 1. Fig. 4 is an overall view of an endoscopic treatment tool 2 inserted into the removal tool 1. Fig. 5 is a view illustrating an internal structure of a distal end portion 2A of the endoscopic treatment tool 2. Fig. 6 is a view illustrating a state in which the endoscopic treatment tool 2 is inserted into the removal tool 1. Fig. 7 is a view illustrating a state in which an adhering substance B (hemoglobin, protein, etc., which is thermally coagulated by cauterization) adhered to an inside of a channel 2B of the endoscopic treatment tool 2 is removed.

The removal tool 1 includes a housing 10, a rod part 20, and a base 30. The housing 10 is a bottomed cylindrical body having an opening at one end, and a hollow portion 12 is formed in the housing 10. The distal end portion 2A of the endoscopic treatment tool 2 is inserted into the hollow portion 12 through an opening 12A of the hollow portion 12 by a worker.

The housing 10 is, for example, a molded resin product, and is formed of, for example, acrylonitrile butadiene styrene (ABS) copolymer or polycarbonate. The housing 10 may be formed of a material through which light having at least a part of visible wavelength is capable of passing, which is, for example, a transparent medium or a translucent medium, so that the worker can view the rod part 20 provided in the hollow portion 12 and the distal end portion 2A of the endoscopic treatment tool 2 inserted in the hollow portion 12. Hereinafter, a "material through which light having at least a part of visible wavelength is capable of passing" is referred to as a "transparent material".

The housing 10 is formed in a cylindrical shape. An axis which is a line on a central axis of the housing 10 is denoted by a reference sign AX. In the following description, an axis AX direction of the housing 10 is defined as a Z direction, and two directions orthogonal to the Z direction and orthogonal to each other are defined as an X direction and a Y direction. The X direction, the Y direction, and the Z direction orthogonal to each other form a left-handed system.

The housing 10 has a cylindrical appearance with a constricted center in a longitudinal direction. Specifically, the housing 10 has an outer shape including a constricted portion 14 having an outer diameter narrower at a central portion than at end portions in the axis AX direction. Since the housing 10 has the outer shape including the constricted portion 14, the worker can easily hold the housing 10 (in other words, the removal tool 1).

The hollow portion 12 of the housing 10 has a shape in which an orthogonal cross section (specifically, an XY cross section) orthogonal to the axis AX direction is circular. The rod part 20 is disposed in the hollow portion 12 having a circular XY cross section.

The rod part 20 is an example of an elongated part to be inserted into the channel 2B of the endoscopic treatment tool 2. The rod part 20 is formed to extend from a bottom 12B of the hollow portion 12 toward the opening 12A side of the hollow portion 12.

Specifically, the rod part 20 is an elongated cylindrical metal component, and is formed of, for example, SUS304. A root of the rod part 20 is embedded in the base 30. The rod part 20 and the base 30 are inserted into a through hole 10B formed in a proximal end portion 10A of the housing 10 and fixed with an adhesive or the like.

The rod part 20 penetrates the through hole 10B and is arranged to extend coaxially with the axis AX in the hollow portion 12 (in other words, it passes through the center of the circular cross section of the hollow portion 12 and along the axis AX direction). In the rod part 20, a portion extending coaxially with the axis AX in the hollow portion 12 is longer than the entire length of the channel 2B of the endoscopic treatment tool 2.

An outer diameter of the rod part 20 is smaller than an inner diameter of the channel 2B of the endoscopic treatment tool 2. The worker, therefore, can insert the rod part 20 into the channel 2B.

Here, the endoscopic treatment tool 2 will be described.

The endoscopic treatment tool 2 is, for example, a treatment tool used for ESD, and includes a high frequency knife 2C at the distal end portion 2A thereof. The amount of protrusion of the high frequency knife 2C with respect to a distal end surface 2Aa of the distal end portion 2A changes depending on a manipulation of an operation unit 2D. The operator manipulates the operation unit 2D depending on a procedure details (marking, local injection, incision, exfoliation, and hemostasis, etc.) and causes the high frequency knife 2C to protrude from the distal end surface 2Aa by an appropriate amount.

In the high frequency knife 2C, the channel 2B that is a through line is formed. The channel 2B is disposed coaxially with the distal end portion 2A having a tubular shape. The channel 2B communicates with a flow path 2E at a subsequent stage. For example, a water supply pump or a suction pump is connected to a proximal end of the flow path 2E.

When the water pump is connected to the proximal end of the flow path 2E, for example, liquid (cleaning liquid, chemical solution, etc.) is supplied into the flow path 2E by the water pump. The liquid supplied into the flow path 2E is injected from the distal end of the high frequency knife 2C to an outside via the flow path 2E and the channel 2B. As a result, for example, it is possible to inject a liquid medicine into a submucosal layer or to remove an adhering substance (blood or mucus) adhered to a body cavity.

When the suction pump is connected to the proximal end of the flow path 2E, for example, the adhering substance adhered to the body cavity is sucked into the channel 2B. The adhering substance sucked into the channel 2B is put into a container or the like included in the suction pump via the flow path 2E.

Hemoglobin, protein, and the like thermally coagulated by cauterization using the high frequency knife 2C may adhere to the channel 2B having such a function. For example, by pumping liquid or gas into the channel 2B using the water pump or the suction pump, it is possible to remove a certain amount of adhering substances from the inside of the channel 2B. However, it is difficult to remove the adhering substance completely solidified in the channel 2B only using such pumping. In addition, the inner diameter of the channel 2B is extremely thin, for example, around 0.2 mm to 0.4 mm. The channel 2B, therefore, is easily clogged with the adhering substances. When the channel 2B is clogged, it becomes difficult to pump liquid or gas into the channel 2B, and it becomes more difficult to remove the adhering substances.

The worker, therefore, removes the adhering substance in the channel 2B using the removal tool 1. A removal work of the adhering substance using the removal tool 1 will be specifically described.

The worker removes the adhering substance in the channel 2B using the removal tool 1 during or after a procedure by the operator. For example, during the procedure, the worker removes the endoscopic treatment tool 2 from an endoscope, and inserts the distal end portion 2A of the endoscopic treatment tool 2 into the hollow portion 12 through the opening 12A of the hollow portion 12 formed in the housing 10.

In the housing 10, slits 16 that divide a side surface 10C of the housing 10 into a plurality of portions are formed in a circumferential direction around the axis AX (here, in a circumferential direction of the housing 10 formed in the cylindrical shape). The slit 16 is formed to extend in the axis AX direction from a distal end portion 10E of the housing 10 toward the proximal end portion 10A to a middle position of the side surface 10C.

For example, the four slits 16 are formed on the side surface 10C of the housing 10 at intervals of 90 degrees in the circumferential direction of the housing 10. As a result, the side surface 10C is divided into four portions in the circumferential direction of the housing 10 at portions where the slits 16 are formed.

The hollow portion 12 includes a tapered portion 12C and a non-tapered portion 12D. The tapered portion 12C is formed to extend in the axis AX direction from the opening 12A to an intermediate position C of the hollow portion 12. The non-tapered portion 12D is formed to extend in the axis AX direction from the intermediate position C, which is the proximal end of the tapered portion 12C, to the bottom 12B.

The tapered portion 12C expands from the bottom 12B side toward the opening 12A. That is, the tapered portion 12C is formed such that the XY cross section orthogonal to the axis AX direction becomes larger from the bottom 12B side toward the opening 12A.

In the first embodiment, the tapered portion 12C has the XY cross section becoming larger continuously from the bottom 12B side toward the opening 12A. In another embodiment, the tapered portion 12C may have an XY cross section becoming larger stepwise from the bottom 12B side toward the opening 12A.

As described above, the hollow portion 12 has a shape in which the XY cross section is circular. Therefore, "expansion" may also be read as "diameter expansion". That is, the tapered portion 12C is formed such that an inner diameter increases continuously or stepwise from the bottom 12B side toward the opening 12A.

The largest XY cross section of the tapered portion 12C (in other words, an inner diameter) is larger than the XY cross section of the distal end portion 2A of the endoscopic treatment tool 2 (in other words, an outer diameter). The worker, therefore, can easily insert the distal end portion 2A into the hollow portion 12 (more precisely, the tapered portion 12C).

The inner diameter of the tapered portion 12C decreases as approaching the non-tapered portion 12D, and becomes smaller than the outer diameter of the distal end portion 2A of the endoscopic treatment tool 2, for example, at a position just before the intermediate position C (the position on the negative side in the Z direction with respect to the intermediate position C). The tapered portion 12C has the smallest inner diameter at the intermediate position C which is the proximal end. The inner diameter of the non-tapered portion 12D is the same as the inner diameter at the intermediate position C over the entire length. That is, the inner diameter of the non-tapered portion 12D is smaller than the outer diameter of the distal end portion 2A of the endoscopic treatment tool 2 over the entire length thereof.

Therefore, when the worker inserts the distal end portion 2A of the endoscopic treatment tool 2 into the tapered portion 12C, an entire peripheral edge portion of the distal end portion 2A of the endoscopic treatment tool 2 is pressed against an inner peripheral surface of the tapered portion 12C near the intermediate position C. In this state, when the worker further inserts the distal end portion 2A toward the bottom 12B, four portions 10D located between the slits 16 in the side surface 10C are elastically deformed such that the inner diameter of the hollow portion 12 is pushed and widened toward the negative side in the Z direction. As a result, the entire hollow portion 12 has a shape enlarged in diameter from the bottom 12B side toward the opening 12A.

That is, the tapered portion 12C further expands in diameter from the bottom 12B side toward the opening 12A, and the non-tapered portion 12D formed as to have a constant inner diameter expands in diameter from the bottom 12B side toward the opening 12A.

Note that a degree of diameter expansion due to elastic deformation of the portion 10D changes depending on the outer diameter of the distal end portion 2A of the endoscopic treatment tool 2 inserted into the hollow portion 12. In the examples of Figs. 6 and 7, the outer diameter of the distal end portion 2A is slightly larger than the inner diameter of the non-tapered portion 12D. Thus, the degree of diameter expansion due to the elastic deformation of the portion 10D is extremely small. Accordingly, the inner diameter of the non-tapered portion 12D is illustrated constant in Figs. 6 and 7 for convenience.

As described above, since the portion 10D is elastically deformed, the worker can insert the distal end portion 2A to a point beyond the intermediate position C (i.e., up to the non-tapered portion 12D). The distal end portion 2A inserted to the non-tapered portion 12D is slidably held in the Z direction at a position coaxial with the axis AX by an external force applied from the entire circumferential direction (specifically, the reaction force from the elastically deformed portion 10D) (see, for example, Fig. 6).

As illustrated in Fig. 6, the channel 2B of the distal end portion 2A held at a position coaxial with the axis AX is located on the same straight line as the rod part 20 similarly extending coaxially with the axis AX. The worker, therefore, can easily insert the rod part 20 into the channel 2B. That is, the removal tool 1 configured as described above has high operability.

Since the housing 10 is formed of a transparent material, the worker can visually grasp the positional relationship between the rod part 20 and the channel 2B. Also from this point, the worker can easily insert the rod part 20 into the channel 2B.

The distal end portion 20A of the rod part 20 has a tapered shape so that the worker can easily insert the rod part 20 into the channel 2B. As illustrated in Fig. 6, the distal end portion 20A has a R chamfered shape. In another embodiment, the distal end portion 20A may be C chamfered or tapered.

An indicator 2F is marked on an outer peripheral surface of the endoscopic treatment tool 2. The indicator 2F indicates an insertion amount of the distal end portion 2A to be inserted into the removal tool 1. As illustrated in Fig. 7, the worker inserts the distal end portion 2A into the hollow portion 12 until the indicator 2F reaches the opening 12A. As a result, the rod part 20 penetrates the channel 2B.

When the rod part 20 penetrates the channel 2B, the adhering substance adhered to the inside of the channel 2B is crushed and pushed out to the flow path 2E. Residues remaining in the channel 2B and the flow path 2E after crushing can be discharged from the channel 2B to the outside by pumping liquid or gas using the water pump or the suction pump.

As described above, according to the first embodiment, the distal end portion 2A of the endoscopic treatment tool 2 inserted into the hollow portion 12 is held coaxially with the rod part 20. The worker, therefore, can easily insert the rod part 20 into the channel 2B.

According to the first embodiment, due to the elastic deformation of the side surface 10C (more specifically, the portion 10D), the inner diameter of the hollow portion 12 changes in accordance with the outer diameter of the distal end portion 2A. The worker, therefore, can use the removal tool 1 for a plurality of types of the endoscopic treatment tools 2 having different outer diameters of the distal end portions 2A.

### [Second Embodiment]

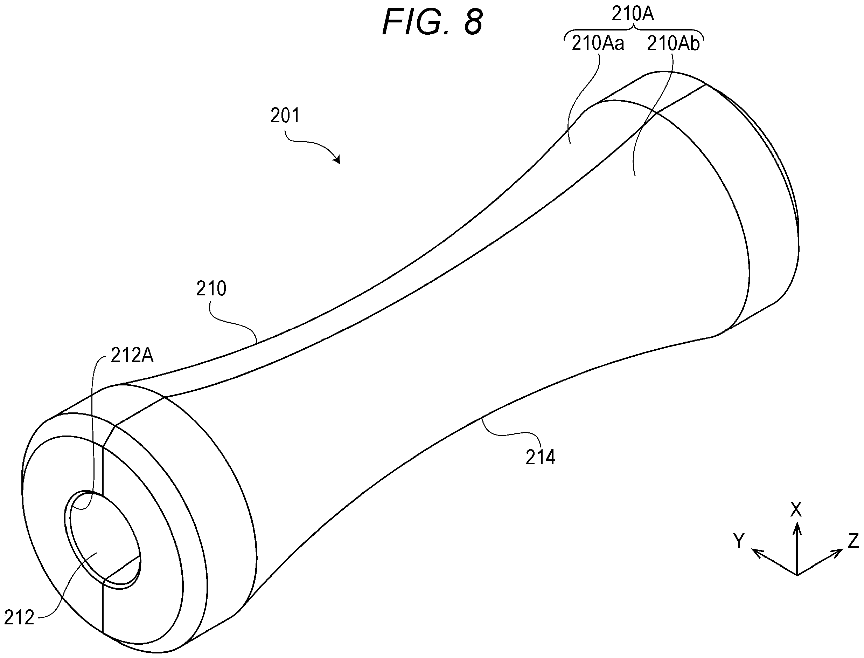

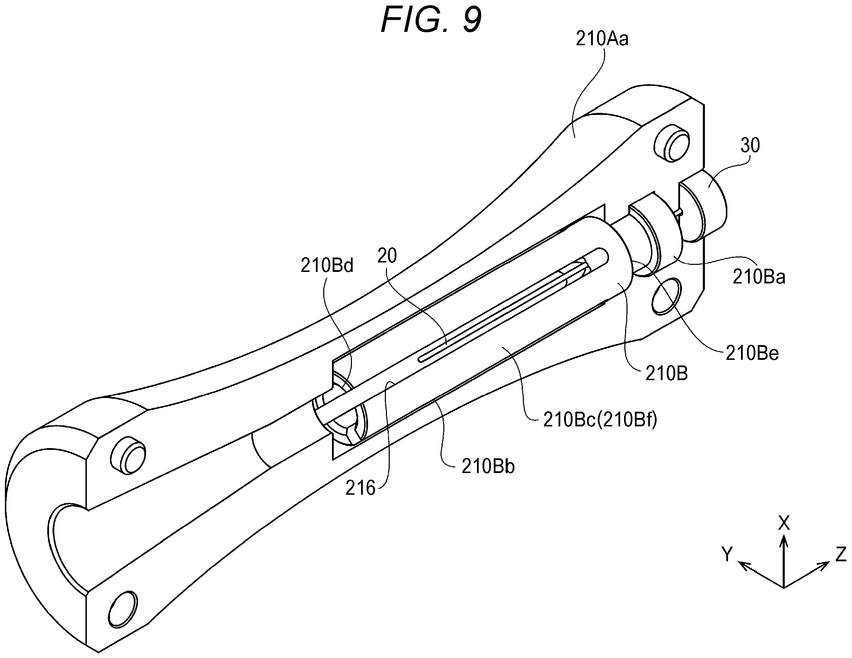

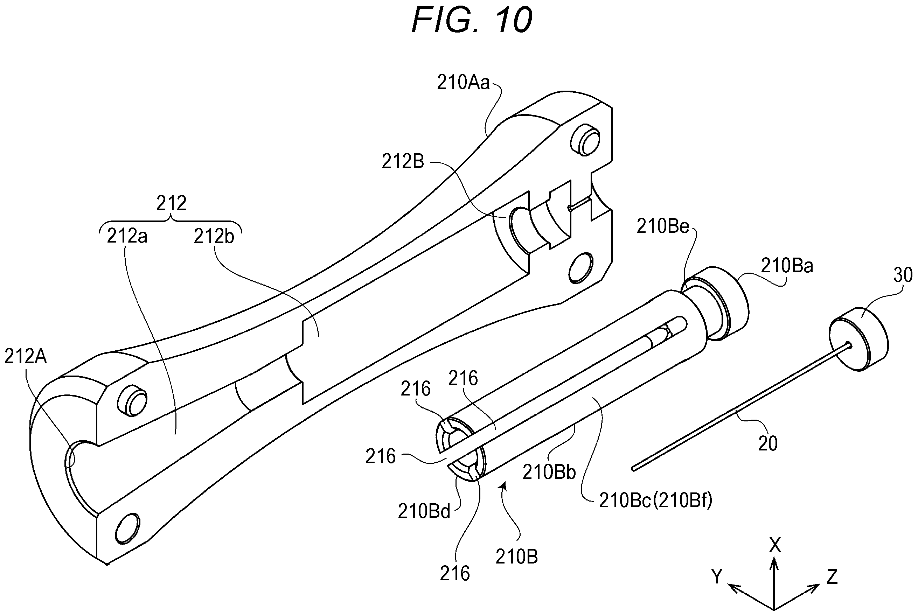

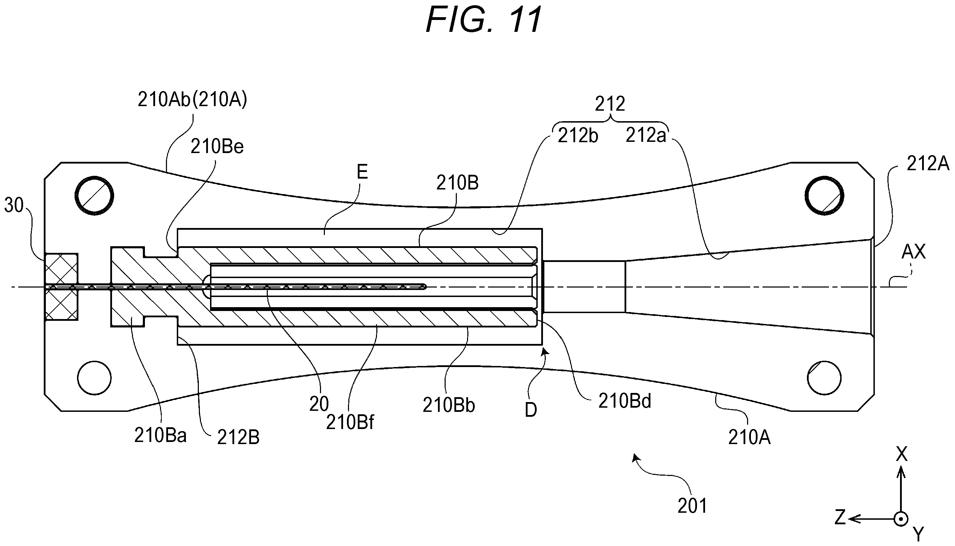

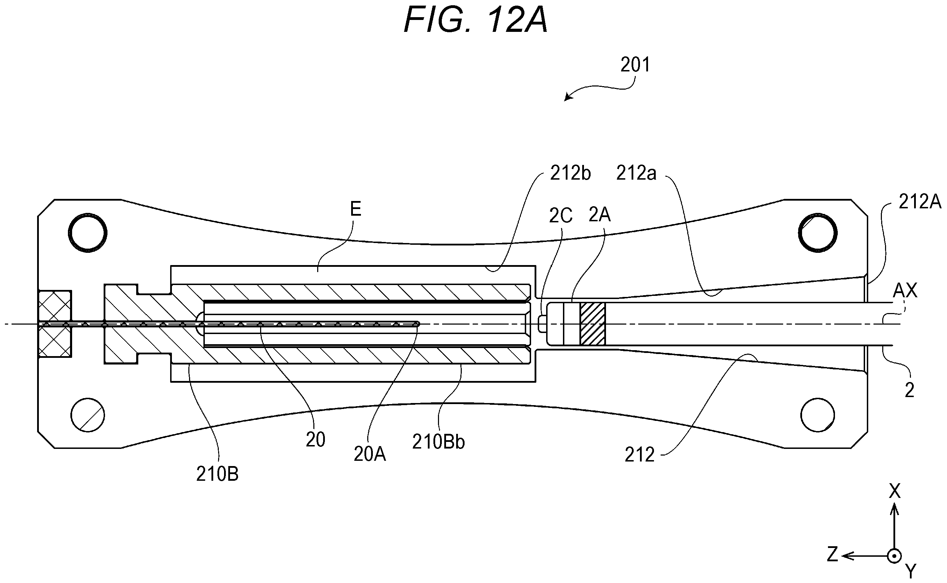

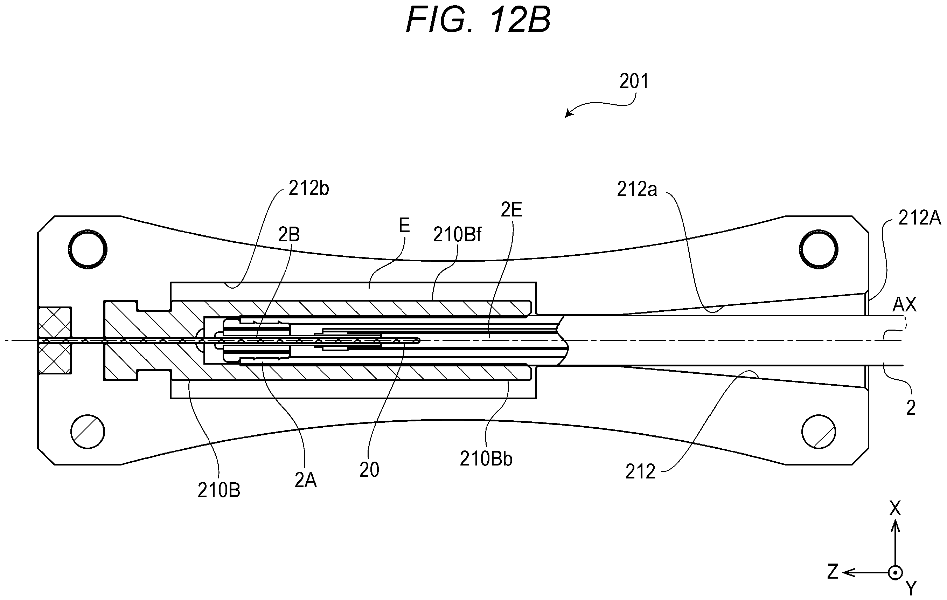

Fig. 8 is a perspective view of a removal tool 201 according to a second embodiment of the present invention. Fig. 9 is a perspective view illustrating an internal structure of the removal tool 201. Fig. 10 is an exploded perspective view illustrating the internal structure of the removal tool 201. Fig. 11 is a cross-sectional view illustrating the internal structure of the removal tool 201. Figs. 12A and 12B are views illustrating states in which the endoscopic treatment tool 2 is inserted into the removal tool 201.

The removal tool 201 includes the rod part 20, the base 30, and a housing 210. The housing 210 includes an exterior part 210A and an inner cylinder part 210B.

The exterior part 210A is, for example, a resin molded product, is a bottomed cylindrical body having an opening at one end, and has a cylindrical appearance (i.e., an outer shape including a constricted portion 214) with a constricted center in a longitudinal direction, similarly to the housing 10 of the first embodiment.

The exterior part 210A includes a pair of halved portions 210Aa and 210Ab. The halved portions 210Aa and 210Ab have a shape obtained by vertically dividing a cylindrical shape in which a center in the longitudinal direction is constricted as described above by a plane including the axis AX direction. By joining the halved portion 210Aa and the halved portion 210Ab at a vertical split surface, the cylindrical exterior part 210A is formed. An illustration of the halved portion 210Ab is omitted In Figs. 9 and 10.

A hollow portion 212 formed in the exterior part 210A includes a guide portion 212a and an accommodating portion 212b. The guide portion 212a is formed to extend in the axis AX direction from an opening 212A of the hollow portion 212 to an intermediate position D of the hollow portion 212. The accommodating portion 212b is formed to extend in the axis AX direction from the intermediate position D, which is a proximal end of the guide portion 212a, to a bottom 212B.

An inner diameter of the guide portion 212a is larger than the outer diameter of the distal end portion 2A of the endoscopic treatment tool 2 over the entire length thereof. The guide portion 212a has a shape expanded in diameter toward the opening 212A. The worker, therefore, can easily insert the distal end portion 2A into the guide portion 212a through the opening 212A (see, for example, Fig. 12A).

The inner cylinder part 210B is accommodated in the accommodating portion 212b. The inner cylinder part 210B is, for example, a resin molded product including a proximal end portion 210Ba and a tubular portion 210Bb. The proximal end portion 210Ba is fixed to the accommodating portion 212b with an adhesive or the like. As a result, the inner cylinder part 210B is disposed coaxially with the exterior part 210A (i.e., at a position centered on the axis AX) in the accommodating portion 212b.

The tubular portion 210Bb is a bottomed cylindrical body having an opening at one end, and is formed in a cylindrical shape. In the tubular portion 210Bb, slits 216 that divide a side surface 210Bc of the tubular portion 210Bb into a plurality of portions in the direction around the axis AX (here, in a circumferential direction of the tubular portion 210Bb formed in a cylindrical shape,) are formed. The slits 216 are formed to extend in the axis AX direction from the distal end portion 210Bd of the tubular portion 210Bb toward the proximal end portion 210Be to an intermediate position of the side surface 210Bc.

For example, four slits 216 are formed on the side surface 210Bc of the tubular portion 210Bb at intervals of 90 degrees in the circumferential direction of the tubular portion 210Bb. As a result, the side surface 210Bc is divided into four portions in the circumferential direction of the tubular portion 210Bb at the portions where the slits 216 are formed.

An inner diameter of the tubular portion 210Bb is smaller than the outer diameter of the distal end portion 2A of the endoscopic treatment tool 2 over the entire length thereof. In addition, there is a clearance (symbol E) between the side surface 210Bc of the tubular portion 210Bb and an inner peripheral surface of the accommodating portion 212b over the entire circumference.

Therefore, when the worker inserts the distal end portion 2A of the endoscopic treatment tool 2 into the tubular portion 210Bb, an entire peripheral edge portion of the distal end portion 2A is pressed against the inner peripheral surface of the tubular portion 210Bb. In this state, when the worker further inserts the distal end portion 2A into the proximal end portion 210Ba, four portions 210Bf located between the slits 216 in the side surface 210Bc are elastically deformed such that the inner diameter of the tubular portion 210Bb expands toward the negative side in the Z direction. As a result, the entire tubular portion 210Bb has a shape enlarged in diameter from the proximal end portion 210Ba side toward the opening 212A.

Since the clearance E having a sufficient size is secured, the elastically deformed portions 210Bf do not collide with the accommodating portion 212b.

In this manner, since the portions 210Bf are elastically deformed, the worker can insert the distal end portion 2A of the endoscopic treatment tool 2 into the tubular portion 210Bb. The distal end portion 2A inserted into the tubular portion 210Bb is slidably held in the Z direction at a position coaxial with the axis AX by external forces applied from the entire circumferential direction (specifically, reaction forces from the elastically deformed portions 210Bf) (see, for example, Fig. 12B).

The channel 2B of the distal end portion 2A held at a position coaxial with the axis AX is located on the same straight line as the rod part 20 also extending coaxially with the axis AX. The worker, therefore, can easily insert the rod part 20 into the channel 2B, crush the adhering substance adhered to the inside of the channel 2B, and push out the adhering substance to the flow path 2E of the endoscopic treatment tool 2. That is, the removal tool 201 configured as described above also has high operability.

As described above, the distal end portion 2A of the endoscopic treatment tool 2 inserted into the hollow portion 212 is held coaxially with the rod part 20 also in the second embodiment. The worker, therefore, can easily insert the rod part 20 into the channel 2B.

The inner diameter of the hollow portion 212 changes in accordance with the outer diameter of the distal end portion 2A due to the elastic deformation of the side surface 210Bc (more specifically, the portions 210Bf) also in the second embodiment. The worker, therefore, can use the removal tool 201 for a plurality of types of the endoscopic treatment tools 2 having different outer diameters of the distal end portions 2A.

The distal end portion 20A of the rod part 20 has a tapered shape so that the worker can easily insert the rod part 20 into the channel 2B also in the second embodiment.

In the second embodiment, by forming the exterior part 210A and the inner cylinder part 210B with a transparent material, the worker can visually grasp the positional relationship between the rod part 20 and the channel 2B. Also from this point, the worker can easily insert the rod part 20 into the channel 2B.

The inner cylinder part 210B, which is an example of the bottomed cylindrical body, has higher pliability than the exterior part 210A. The worker, therefore, can insert the distal end portion 2A into the inner cylinder part 210B with a light load. In addition, since the exterior part 210A itself gripped by the worker is not elastically deformed, the worker can easily handle the removal tool 201.

The exterior part 210A and the inner cylinder part 210B are formed of, for example, the same material. The inner cylinder part 210B is formed to be thinner than the exterior part 210A, and thus has higher pliability than the exterior part 210A.

The exterior part 210A and the inner cylinder part 210B may be formed of different materials. As an example, the inner cylinder part 210B is formed of a material having a lower elastic modulus than the exterior part 210A.

### [Third Embodiment]

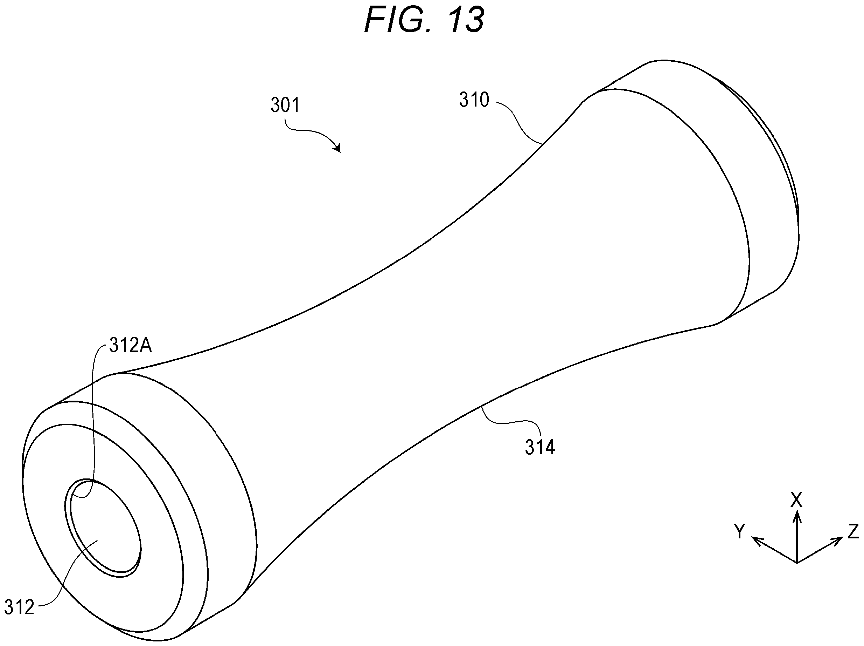

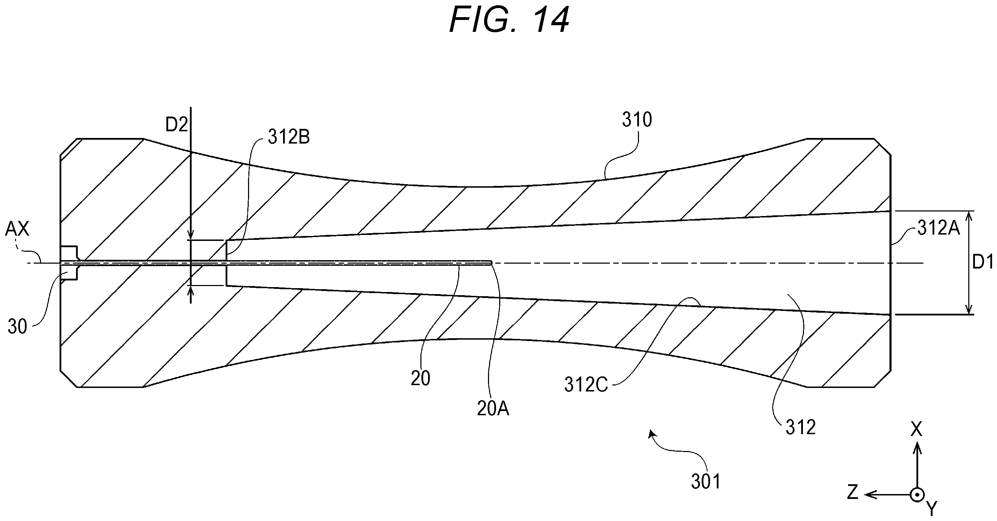

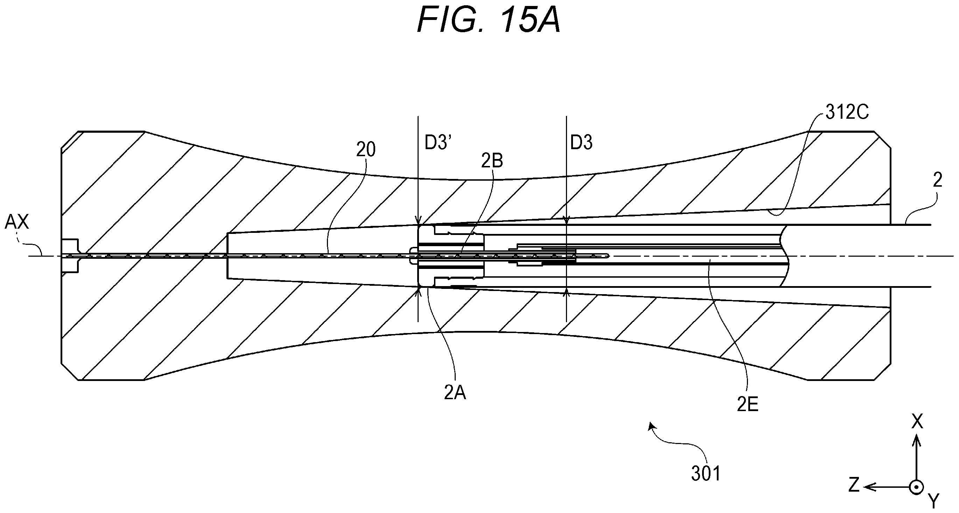

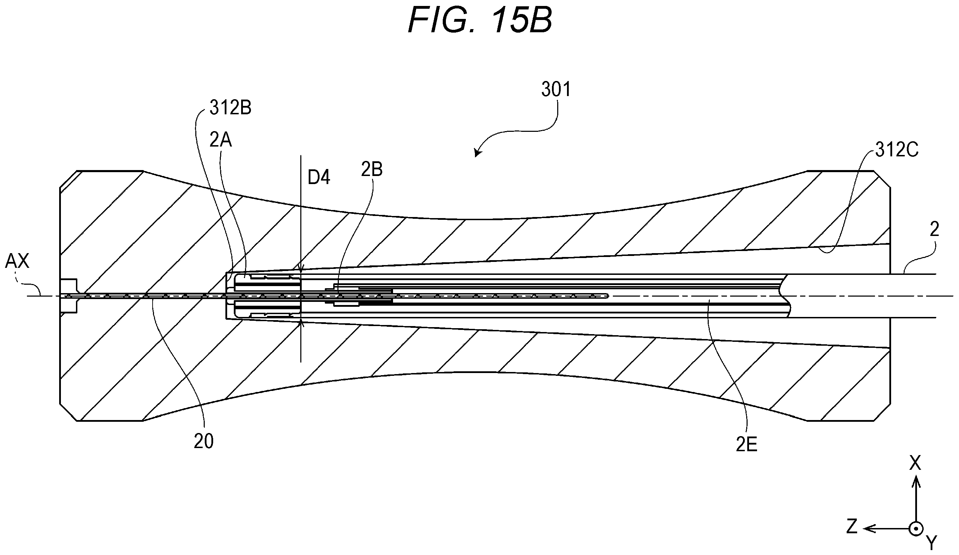

Fig. 13 is a perspective view of a removal tool 301 according to a third embodiment of the present invention. Fig. 14 is a cross-sectional view illustrating an internal structure of the removal tool 301. Figs. 15A and 15B are views illustrating a state in which the endoscopic treatment tool 2 is inserted into the removal tool 301.

The removal tool 301 includes the rod part 20, the base 30, and a housing 310. The housing 310 is, for example, a resin molded product, is a bottomed cylindrical body having an opening at one end, and has a cylindrical appearance (i.e., an outer shape including a constricted portion 314) with a constricted center in a longitudinal direction, similarly to the housing 10 of the first embodiment.

The housing 310 is different from the housing 10 of the first embodiment in which no slit is formed. Instead, a tapered portion 312C is formed in a hollow portion 312 of the housing 310.

In the third embodiment, the tapered portion 312C ranges over an entire portion from a bottom 312B of the hollow portion 312 to an opening 312A. The tapered portion 312C has a truncated cone shape expanding in diameter from the bottom 312B side toward the opening 312A. That is, the tapered portion 312C has the largest inner diameter at a position on the most negative side in the Z direction and the smallest inner diameter at a position on the most positive side in the Z direction.

In this manner, the inner diameter of the tapered portion 312C increases continuously from the bottom 312B side toward the opening 312A. On the other hand, a configuration in which the inner diameter of the tapered portion 312C increases stepwise from the bottom 312B side toward the opening 312A is also included in a scope of the present invention.

Hereinafter, a maximum inner diameter of the tapered portion 312C is denoted by a reference numeral D1, and a minimum inner diameter of the tapered portion 312C is denoted by a reference numeral D2.

In an example of Fig. 15A, the distal end portion 2A having an outer diameter D3 is inserted into the tapered portion 312C. The outer diameter D3 is smaller than the inner diameter D1 of the tapered portion 312C and larger than the inner diameter D2 of the tapered portion 312C.

Since the outer diameter D3 is smaller than the inner diameter D1, the worker can insert the distal end portion 2A of the endoscopic treatment tool 2 into the tapered portion 312C. The worker can insert the rod part 20 into the channel 2B by further inserting the distal end portion 2A.

The tapered portion 312C has the same inner diameter as the outer diameter D3 at an intermediate position D3'. Therefore, the entire peripheral edge portion of the distal end portion 2A of the endoscopic treatment tool 2 is pressed against an inner peripheral surface of the tapered portion 312C at the intermediate position D3'. When the distal end portion 2A is inserted to the intermediate position D3', the rod part 20 penetrates the channel 2B. As a result, the adhering substance adhered to the inside of the channel 2B is crushed and pushed out to the flow path 2E of the endoscopic treatment tool 2.

When the distal end portion 2A of the endoscopic treatment tool 2 is pressed against the inner peripheral surface of the tapered portion 312C and stopped, the worker can grasp that the rod part 20 has penetrated the channel 2B (in other words, the adhering substance is crushed and pushed out to the flow path 2E of the endoscopic treatment tool 2). In addition, the worker can remove the adhering substance in the channel 2B without inserting the distal end portion 2A to the deepest portion of the tapered portion 312C (here, by simply inserting the distal end portion 2A to the intermediate position D3'). As described above, the removal tool 301 according to the third embodiment also has high operability.

In the example of Fig. 15B, the distal end portion 2A having an outer diameter D4 is inserted into the tapered portion 312C. The outer diameter D4 is smaller than the inner diameter D2 of the tapered portion 312C. The worker, therefore, can insert the distal end portion 2A to the bottom 312B which is the deepest portion of the tapered portion 312C.

When the distal end portion 2A is inserted to the bottom 312B, the rod part 20 penetrates the channel 2B. As a result, the adhering substance adhered to the inside of the channel 2B is crushed and pushed out to the flow path 2E of the endoscopic treatment tool 2.

When the distal end portion 2A of the endoscopic treatment tool 2 is pressed against the bottom 312B of the tapered portion 312C and stopped, the worker can grasp that the rod part 20 has penetrated the channel 2B (in other words, the adhering substance is crushed and pushed out to the flow path 2E of the endoscopic treatment tool 2).

In the third embodiment, the removal tool 301 can be used for a plurality of types of endoscopic treatment tools 2 in which the outer diameters of the distal end portions 2A are smaller than the inner diameter D1.

When the distal end portion 2A having an outer diameter larger than the inner diameter D2 and smaller than the inner diameter D1 is inserted into the tapered portion 312C, the entire peripheral edge portion of the distal end portion 2A is pressed against the inner peripheral surface of the tapered portion 312C at an intermediate position in the tapered portion 312C. In this case, the distal end portion 2A cannot move in the tapered portion 312C unless the worker moves the distal end portion 2A to the negative side in the Z direction. By perceiving that the distal end portion 2A cannot be moved in the tapered portion 312C, the worker can more clearly grasp that the rod part 20 has penetrated the channel 2B.

The housing 310 is formed of a transparent material. The worker, therefore, can visually grasp the positional relationship between the rod part 20 and the channel 2B. Also from this point, the worker can easily insert the rod part 20 into the channel 2B.

The distal end portion 20A of the rod part 20 has a tapered shape so that the worker can easily insert the rod part 20 into the channel 2B also in the third embodiment.

### [Fourth Embodiment]

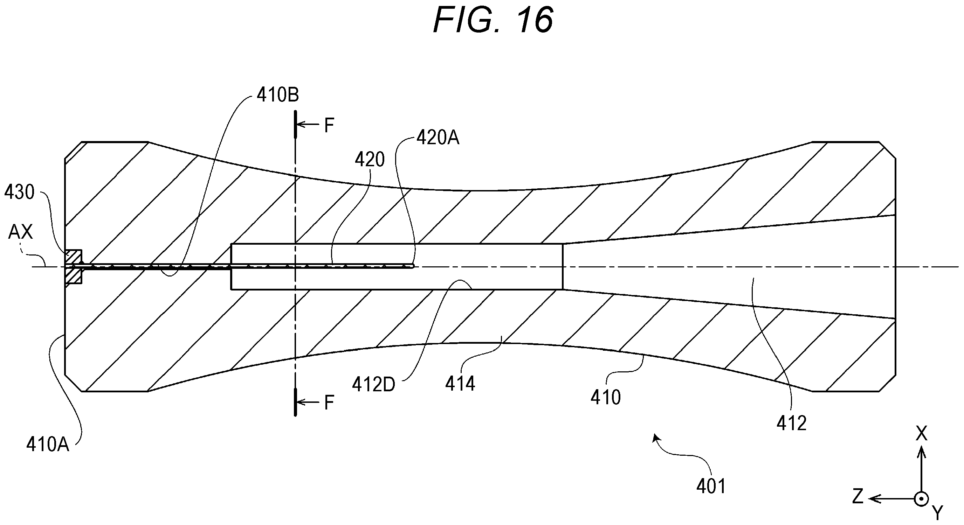

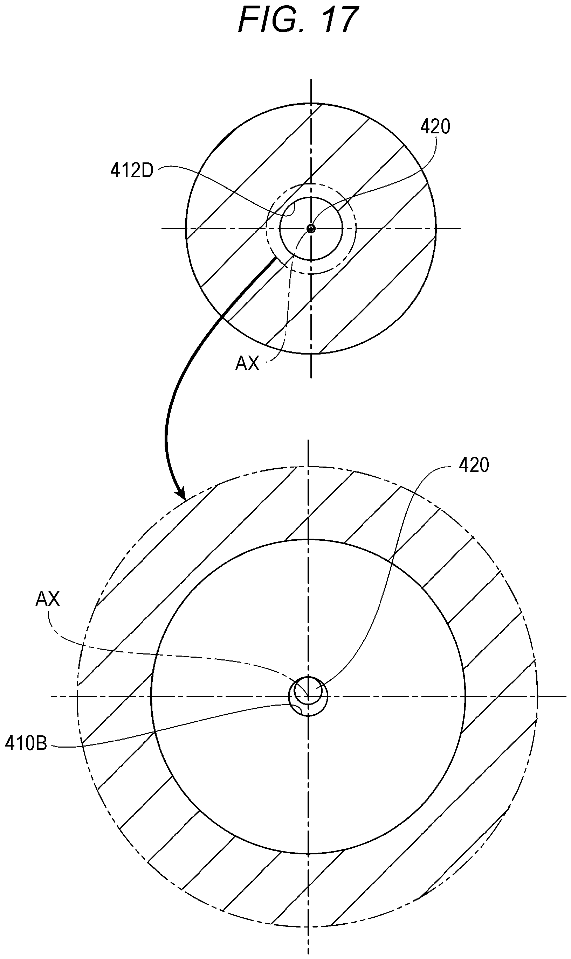

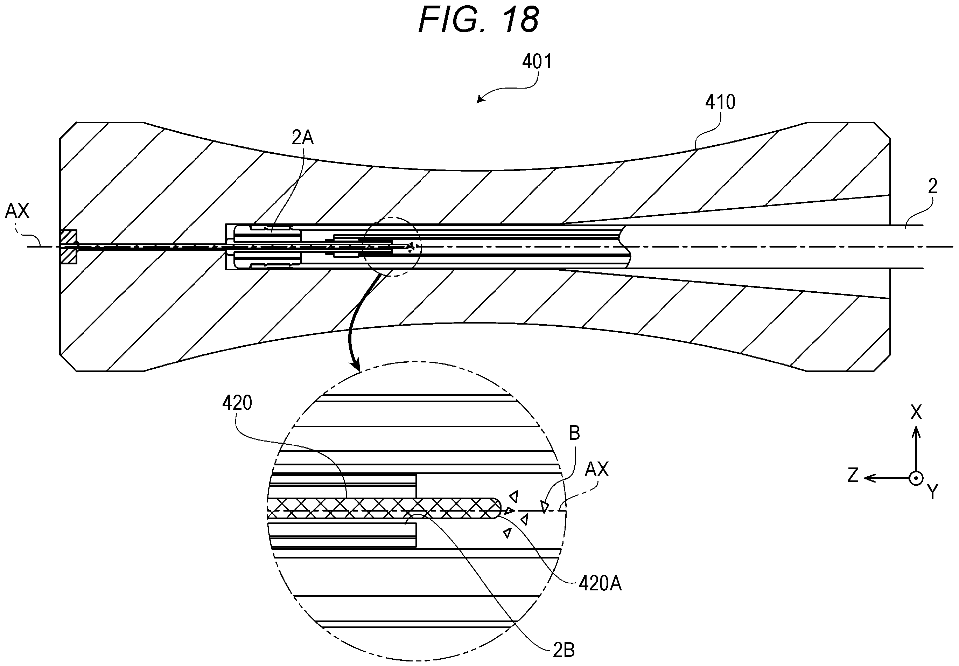

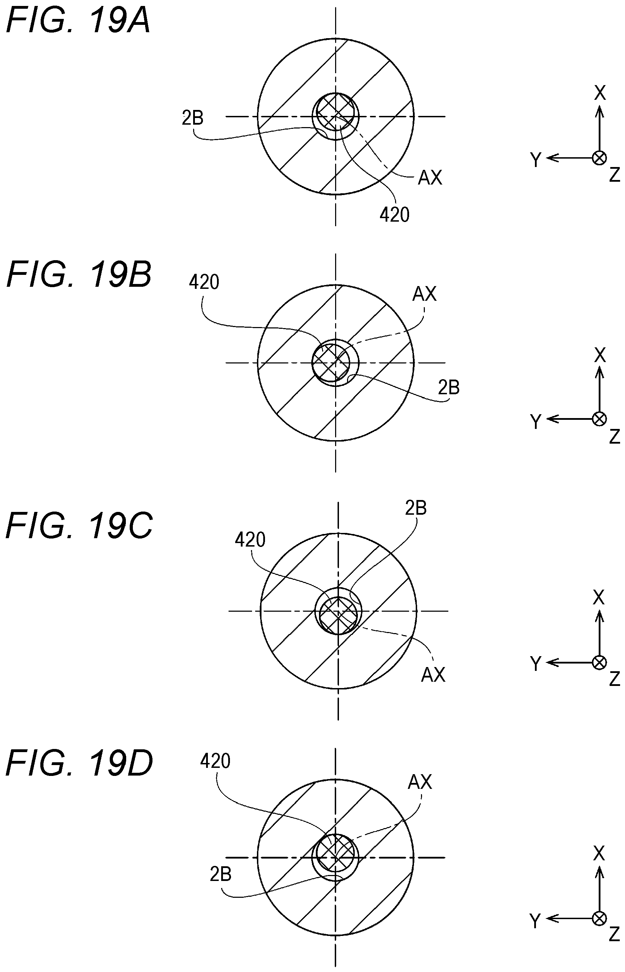

Fig. 16 is a cross-sectional view illustrating an internal structure of a removal tool 401 according to a fourth embodiment of the present invention. Fig. 17 is a cross-sectional view taken from line F-F in Fig. 16. Fig. 18 is a view illustrating a state in which the endoscopic treatment tool 2 is inserted into the removal tool 401. Figs. 19A to 19D are cross-sectional views illustrating a state in which the endoscopic treatment tool 2 is inserted into the removal tool 401.

The removal tool 401 includes a housing 410, a rod part 420, and a base 430. The housing 410 is, for example, a resin molded product, is a bottomed cylindrical body having an opening at one end, and has a cylindrical appearance with a constricted center in a longitudinal direction (i.e., an outer shape including a constricted portion 414), similarly to the housing 10 of the first embodiment. The housing 410 is the same as the housing 10 of the first embodiment except that the slits 16 are not formed.

The base 430 has a cylindrical shape. A root of the rod part 420 is embedded at a position shifted from a center of the base 430 (eccentric position). The rod part 420 and the base 430 are inserted into a through hole 410B formed in a proximal end portion 410A of the housing 410 and fixed with an adhesive or the like. As a result, the rod part 420 penetrates the through hole 410B and is arranged to extend at a position eccentric to the axis AX in a hollow portion 412 of the housing 410 (in other words, it passes through a position deviated from a center of a circular cross section of the hollow portion 412 and extends along the axis AX direction).

In the fourth embodiment, the outer diameter of the distal end portion 2A of the endoscopic treatment tool 2 and an inner diameter of a non-tapered portion 412D of the hollow portion 412 are substantially the same. Therefore, when the distal end portion 2A is inserted into the non-tapered portion 412D, the distal end portion 2A is positioned coaxially with the axis AX which is a central axis of the non-tapered portion 412D.

On the other hand, the rod part 420 is located eccentrically from the axis AX. An outer diameter of the rod part 420 is smaller than the inner diameter of the channel 2B. When the distal end portion 2A is inserted into the non-tapered portion 412D, as illustrated in Fig. 19A, the rod part 420 penetrates the inside of the channel 2B in a state of being in contact with the inner peripheral surface of the channel 2B at a position eccentric from the axis AX.

Therefore, when the worker rotates the removal tool 401 about the axis AX with respect to the distal end portion 2A, as illustrated in Figs. 19A to 19D, the rod part 420 moves at the position eccentric to the axis AX in the channel 2B so as to rub the inner peripheral surface of the channel 2B. As a result, the adhering substance adhered to the inside of the channel 2B is scraped off by the rod part 420 and crushed. Therefore, the adhering substance can be reliably removed from the inside of the channel 2B.

An edge portion may be formed on a side surface of the rod part 420.

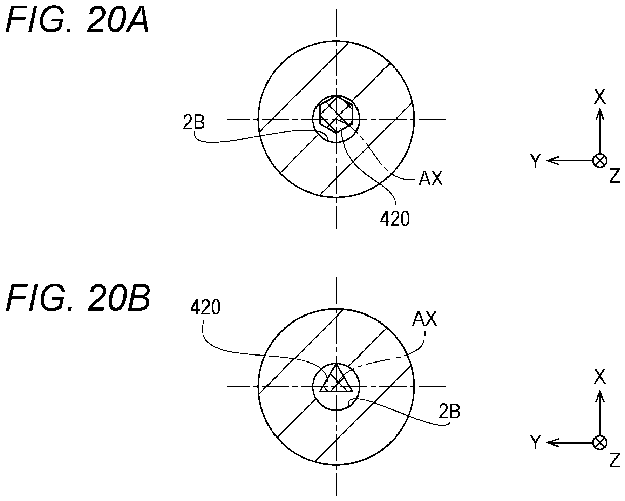

Fig. 20A illustrates an XY cross section of a rod part 420 according to a first modification. In the example of Fig. 20A, the rod part 420 is formed to have a hexagonal XY cross section, and has a shape including a total of six edge portions on the side surface.

Fig. 20B illustrates an XY cross section of a rod part 420 according to a second modification. In the example of Fig. 20B, the rod part 420 is formed to have a triangular XY cross section, and has a shape including a total of three edge portions on the side surface.

In the examples of Figs. 20A and 20B, when the worker rotates the removal tool 401 about the axis AX with respect to the distal end portion 2A, the edge portions of the rod part 420 rub the inside of the channel 2B. Since the inside of the channel 2B can be strongly rubbed by the edge portions in line contact with the inner peripheral surface of the channel 2B, the adhering substance can be more reliably removed from the inside of the channel 2B.

A distal end portion 420A of the rod part 420 has a tapered shape so that the worker can easily insert the rod part 420 into the channel 2B also in the fourth embodiment.

The housing 410 is formed of a transparent material. The worker, therefore, can visually grasp the positional relationship between the rod part 420 and the channel 2B. As a result, the worker can easily insert the rod part 420 into the channel 2B.

Exemplary embodiments of the present invention are described above. The embodiments of the present invention are not limited to those described above, and various modifications can be made within the scope of the technical idea of the present invention. For example, contents in appropriate combination with the embodiments illustratively clarified in the description or obvious embodiments are also included in the embodiments of the present invention.

As an example of contents obtained by appropriately combining the embodiments and the like, a configuration in which an indicator 2F is marked on the outer peripheral surface of the endoscopic treatment tool 2 is exemplified in the second to fourth embodiments similarly to the first embodiment.

As an example of contents obtained by appropriately combining the embodiments and the like, a configuration in which an edge portion is formed on a side surface of the rod part 20 is exemplified in the first to third embodiment similarly to first and second modifications of the fourth embodiment.

## Claims

1. A removal tool for removing an adhering substance adhered to an inside of a channel of an endoscopic treatment tool, the removal tool comprising:
a bottomed cylindrical body having an opening at one end; and
an elongated part formed in a hollow portion of the bottomed cylindrical body so as to extend from a bottom of the hollow portion toward an opening side of the hollow portion, the elongated part being capable of inserting into the channel, wherein
the hollow portion expands from the bottom side toward the opening.

2. The removal tool according to claim 1, wherein
a plurality of slits dividing a side surface of the bottomed cylindrical body into a plurality of portions are formed in a circumferential direction around an axis of the bottomed cylindrical body, and
when the endoscopic treatment tool is inserted into and pressed against the hollow portion, a portion of the bottomed cylindrical body located between the slits is elastically deformed, which causes the hollow portion to expand from the bottom side toward the opening.

3. The removal tool according to claim 2, further comprising an exterior part holding the bottomed cylindrical body, wherein
the bottomed cylindrical body has higher pliability than the exterior part.

4. The removal tool according to claim 1, wherein
the hollow portion includes a tapered portion expanding continuously or stepwise from the bottom side toward the opening.

5. The removal tool according to any one of claims 1 to 4, wherein
the bottomed cylindrical body includes
a constricted portion formed in a cylindrical shape and having an outer diameter narrower at a central portion than at end portions in an axial direction of the bottomed cylindrical body.

6. The removal tool according to any one of claims 1 to 4, wherein
the bottomed cylindrical body is formed of a material through which light having at least a part of visible wavelength is capable of passing.

7. The removal tool according to any one of claims 1 to 4, wherein
an edge portion is formed on a side surface of the elongated part.

8. The removal tool according to any one of claims 1 to 4, wherein
a distal end portion of the elongated part has a tapered shape.

9. The removal tool according to any one of claims 1 to 4, wherein
the hollow portion has a circular cross section orthogonal to an axial direction of the bottomed cylindrical body, and
the elongated part is disposed in the hollow portion so as to pass through a center of the circular cross section and extend along the axial direction.

10. The removal tool according to any one of claims 1 to 4, wherein
the hollow portion has a circular cross section orthogonal to an axial direction of the bottomed cylindrical body, and
the elongated part is disposed in the hollow portion so as to pass through a position deviated from a center of the circular cross section and extend along the axial direction.

11. A removal system comprising:
the removal tool according to any one of claims 1 to 4; and
an endoscopic treatment tool to be inserted into the removal tool, wherein
an indicator indicating an amount of the endoscopic treatment tool to be inserted into the removal tool is marked on the endoscopic treatment tool.

12. A removal tool for removing an adhering substance adhered to an inside of a channel of an endoscopic treatment tool, the removal tool comprising:
a bottomed cylindrical body having an opening at one end; and
an elongated part formed in a hollow portion of the bottomed cylindrical body so as to extend from a bottom of the hollow portion toward an opening side of the hollow portion, the elongated part being capable of inserting into the channel, wherein
the hollow portion has a circular cross section orthogonal to an axial direction of the bottomed cylindrical body, and
the elongated part is disposed in the hollow portion so as to pass through a position deviated from a center of the circular cross section and extend along the axial direction.

13. The removal tool according to claim 12, wherein
the bottomed cylindrical body includes
a constricted portion formed in a cylindrical shape and having an outer diameter narrower at a central portion than at end portions in an axial direction of the bottomed cylindrical body.

14. The removal tool according to claim 12 or 13, wherein
the bottomed cylindrical body is formed of a material through which light having at least a part of visible wavelength is capable of passing.

15. The removal tool according to claim 12 or 13, wherein
an edge portion is formed on a side surface of the elongated part.

16. The removal tool according to claim 12 or 13, wherein
a distal end portion of the elongated part has a tapered shape.

17. A removal system comprising:
the removal tool according to any one of claim 12 or 13; and
an endoscopic treatment tool to be inserted into the removal tool, wherein
an indicator indicating an amount of the endoscopic treatment tool to be inserted into the removal tool is marked on the endoscopic treatment tool.
